Europäisches Patentamt

(19) European Patent Office

Office européen des brevets

(11) Publication number: **0 038 110**
**B1**

(12) **EUROPEAN PATENT SPECIFICATION**

(45) Date of publication of patent specification: **23.05.84**

(51) Int. Cl.³: **A 61 N 1/06,** H 05 K 9/00

(21) Application number: **81300455.3**

(22) Date of filing: **03.02.81**

(54) Diathermy applicator head incorporating electrostatic shields.

(30) Priority: **31.03.80 US 136101**

(43) Date of publication of application:
**21.10.81 Bulletin 81/42**

(45) Publication of the grant of the patent:
**23.05.84 Bulletin 84/21**

(84) Designated Contracting States:
**BE CH DE FR GB IT LI NL SE**

(56) References cited:
**DE-C- 718 637**
**DE-C- 937 431**
**GB-A- 390 500**
**US-A-4 068 292**

(73) Proprietor: **INTERNATIONAL MEDICAL ELECTRONICS, LTD.**
**2805 Main Street**
**Kansas City Missouri 64108 (US)**

(72) Inventor: **Berry, Frederick Marion**
**10101 Wenonga Lane**
**Leawood Kansas 66206 (US)**

(74) Representative: **Jennings, Roy Alfred et al**
**GILL JENNINGS & EVERY 53-64 Chancery Lane**
**London WC2A 1HN (GB)**

Courier Press, Leamington Spa, England.

## Description

This invention relates to a diathermy applicator head for a short wave diathermy apparatus, the head comprising a current carrying radiating coil, an electrostatic shield which includes a plurality of electrically conductive first strips which are located so that, in use, they are interposed between the coil and the body of a patient being treated by the coil and a plurality of further conductive strips which surround the coil, the first strips extending inwards from the further strips, and means for earthing the shield.

Diathermy treatments involve the application of high frequency electric currents to body tissues. This technique utilizes the transcutaneous transmission of high frequency energy to the internal body tissues to be treated. The energy employed is sufficiently high to prevent adverse stimulation of nerves and muscles and is sufficiently low to prevent destruction of the treated tissue.

In a conventional diathermy apparatus, radio frequency electric currents are generated by the apparatus. These high frequency electric currents are then applied to the applicator head of the apparatus and the applicator head converts the high frequency currents into electromagnetic and electrostatic energy. The generated electromagnetic and electrostatic energy is then applied in a controlled manner by the applicator head to the body of the patient being treated. This energy causes heat to be generated in the internal body tissues which are within the radiating range of the applicator head.

Painful heating of the subcutaneous fat layer and irritating skin burns may occur when a patient is treated using conventional diathermy apparatus. It has been found that the deleterious surface heating effects just described are primarily caused by the electrostatic field radiated by conventional diathermy applicator heads. The associated radiated electromagnetic field, however, does not cause the above-mentioned harmful burning of surface tissues. Furthermore, the radiated electromagnetic field penetrates deeply enough to provide relative heating in the muscle tissues without painful burning of the subcutaneous fat layer and skin tissue. This deep heating is at least partially produced because the electromagnetic field lines are tangential to the tissue interfaces rather than perpendicular and, as a result, the boundary conditions do not significantly produce or cause surface heating effects. As a result, the therapeutic deep heating benefits of diathermy treatment are primarily caused by the electromagnetic energy radiated by the applicator head.

By attenuating the electrostatic field radiated by the applicator head before it reaches the patient being treated, it is possible virtually to eliminate the above-described surface heating effects. A technique and apparatus for accomplishing this result are described in U.S.—A—4,068,292. The shield disclosed in this Patent comprises a plurality of non-magnetic metallic (preferably copper) strips which are located within a coplanar, generally circular loop. Each of the strips is maintained at a generally perpendicular orientation with respect to the current carrying coil in the diathermy applicator head on which the shield is mounted. Each of the strips extends generally radially outwardly from the vicinity of the centre of the loop and terminates at and is electrically connected to the circular loop at its outer end.

Another form of diathermy applicator head provided with an electrostatic shield is disclosed in DE—C—937,431. This head comprises a current carrying radiating coil and a shield which comprises a plurality of electrically conductive strips each of which extends across a plane between the coil and the body of a patient being treated and which has both ends bent so that they extend beside the coil, the free ends of the bent parts of the strips then being earthed to a housing of the head.

It has been found that existing applicator heads sometimes induce circulating or eddy currents in the circular loop of the shield of the type disclosed in U.S.—A—4,068,292.

In many instances the creation of circulating or eddy currents in the loop of the shield or the outer casing of the applicator head is highly undesirable and may be responsible for several detrimental effects. In particular circulating currents in the applicator head casing produces an associated power loss which significantly reduces the operating efficiency of the diathermy apparatus. Another problem associated with such circulating currents within the outer casing of the applicator head is that the casing becomes extremely hot in response to such current flow therein. In fact, the outer casing of the head can become so hot that it is impossible to touch the head while the apparatus is in use. Accordingly, the minimization of such circulating currents in the outer casing of the applicator head or in the circular loop of the associated shield is highly desirable.

The shield disclosed in DE—C—937,431, on the other hand, tends, if the strips are sufficiently close together to act as an effective electrostatic shield, to shield also the electromagnetic field and thus decrease the effectiveness of the head. This is because the strips extend wholly across the plane between the coil and the body of the patient.

The object of the present invention is to provide an applicator head of a shortwave diathermy apparatus, the head being provided with a shield which overcomes the aforementioned problems and reduces the intensity of the circulating or eddy currents induced in the components of the shield or in the casing of the applicator head. In consequence, the power loss in the applicator head is also reduced and the operating efficiency of the diathermy apparatus is improved.

To this end, according to this invention, a diathermy applicator head as initially described is characterized in that the coil is substantially circular and planar, the first strips extend in a plane substantially parallel to the plane of the coil, but leave an open centre area, and an electrically conductive loop electrically connects the ends of the further strips remote from the first strips, the loop being spaced axially from the coil and on the side thereof remote from the first strips by a distance at least as great as the diameter of the coil so that only negligible electric currents are induced in the loop when current is applied to the coil.

The further strips form a side portion of the shield encircling the coil of the applicator head. The side portion of the shield together with the loop form a closed structure having a hollow interior. The wall of the structure is slotted to provide the further plurality of axially extending strips.

As a result of the first strips which form the front portion of the shield extending radially inwards from the periphery of the front so as to cover a major part of the surface area defined by the front portion of the shield with each of these strips electrically coupled with one of the further plurality of axially extending strips which make up the side portion of the shield, each axial strip and its associated face strip or strips act as a single continuous strip which extend axially and which terminate within the area formed by the face portion of the shield. Further, all these strips are interconnected in a position remote from the coil by the conductive loop.

As a result of this arrangement, the electrostatic shield provides improved attenuation of the electrostatic field which is radiated by the radiating coil of the applicator head. A primary benefit to be derived from the attenuation of the electrostatic field resides in the significant improvement in the operating efficiency of the diathermy apparatus. In particular, greater attenuation of the electrostatic field by the electrostatic shield serves to reduce electrostatic (capacitive) coupling between the treated body tissues and the applicator head. The reduction or elimination of capacitive coupling between the applicator head and body tissues stabilizes the operation of the diathermy apparatus and thereby reduces the likelihood that the applicator will be detuned from resonance upon an introduction of a load into the radiating region of the coil of the applicator head. By stabilizing the operation of the diathermy apparatus, it becomes easier to keep the applicator head in electrical resonance thereby improving the accuracy of the power output made by the apparatus.

Use of the shield also enhances the operation of the diathermy apparatus by reducing head loss. The electrostatic shield serves to isolate the electrostatic field radiated by the radiating coil in the applicator head from a loop portion of the shield and from an outer casing of the applicator head when such a casing is provided. By isolating the electrostatic field in this manner, the creation of circulating or eddy currents within the loop portion of the shield and in the outer casing of the applicator head is significantly reduced thereby improving the operating efficiency of the head.

An example of a diathermy applicator head incorporating an electrostatic shield in accordance with the invention is illustrated in the accompanying drawings in which:—

Figure 1 is a partly sectional plan view of the applicator head showing part of the shield within it; and,

Figure 2 is a sectional view of the applicator head and shield taken along the line 2—2 of Figure 1.

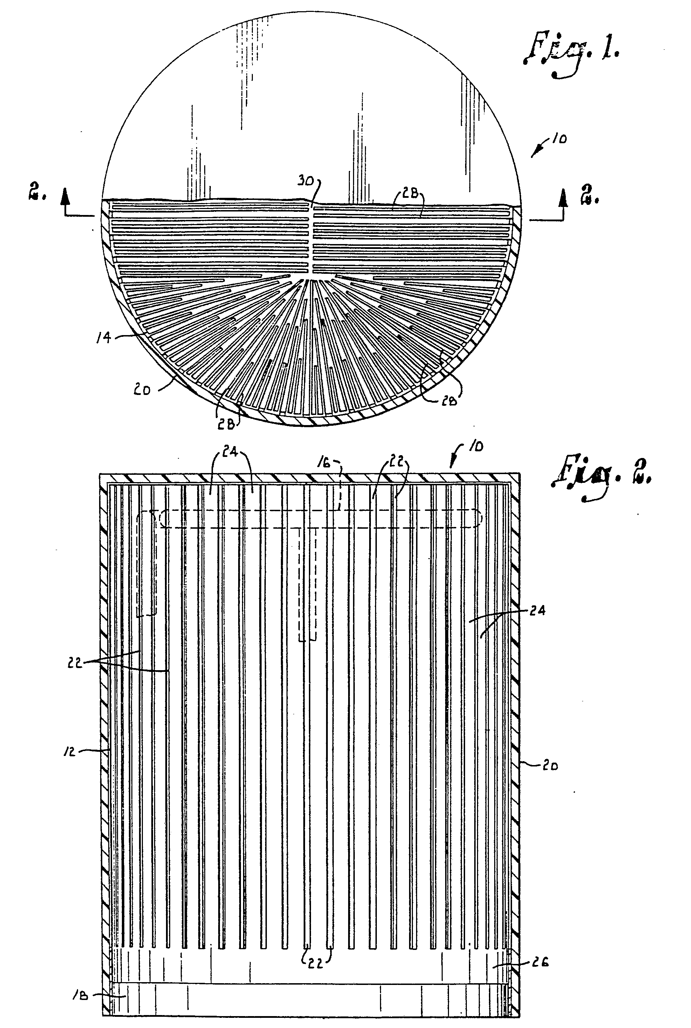

Referring to Figures 1 and 2 of the drawings, an electrostatic shield 10, shown in these figures, is constructed for use in an applicator head having a circular configuration and as a result comprises a cylindrically shaped side portion 12 and a face portion 14. The electrostatic shield 10 of the present invention is arranged to be mounted to a diathermy applicator head such that the face portion of the shield is in a substantially parallel relationship to the plane formed by the current carrying coil in the applicator head. Such a current carrying coil is shown in Figure 2 in broken lines and is designated by the numeral 16. The applicator head also includes a metal base plate 18 and a rigid non-conductive outer casing 20. The casing 20 may be eliminated if the shield is sufficiently rigid.

It should be noted at this time that the disclosure of an electrostatic shield for use on a "circular" applicator head is merely illustrative and should not be interpreted in a limiting sense. In fact, the configuration of the shield may be changed to accommodate an applicator head having any desired configuration. This modification is made by constructing the face portion of the shield to have a configuration which conforms to the shape of the applicator head and then extending the strips which form the side portion of the shield downward around the radiating electrodes in the head from the outer periphery of the face portion of the shield.

The side portion 12 of the shield is comprised of a copper cylinder having a plurality of slots (such as 22) cut therein to provide a plurality of axial strips 24. The slots 22 cut in the side portion of the shield extend from the outer ridge of the side portion to a point just above the inner edge of the side portion so as to form a terminating band or loop 26.

In the preferred embodiment of the invention, the side portion of the shield is constructed to have a length at least equal to the diameter of the heads radiating coil 16. In fact, it is sometimes desirable to extend the length of the side portion of the shield even longer to thereby decrease the magnitude of the circulating currents induced within the loop by the electromagnetic field radiated by the current carrying coil of the applicator head. While it is desirable to position this loop as far away from the coil as possible, it is impractical to make the head too long and the magnitude of the circulating currents is negligible if the shield is constructed so that the distance between the terminating band 26 and the coil 16 is at least equal to the diameter of the coil. In addition, the face portion of the shield is spaced away from the current carrying coil 16 of the head by a distance sufficient to prevent arcing between the coil and the shield.

The face portion 12 of shield 10 is comprised of a plurality of electrically conductive strips such as 28 (preferably made of copper) which extend radially inward from the outer periphery of the face portion's surface. These electrically conductive strips 28 are affixed to a non-metallic mounting surface 20 by adhesion or by metalizing same on the non-metallic mounting surface by conventional etching techniques.

As shown in Figure 1, the strips 28 which make up the face portion of the shield extend radially inward from the outer periphery of the surface defined by the face portion of the shield towards the open centre area 30 of the shield. It is significant to note that the width of the strips is very small in proportion to the wave length of the energy emitted by the associated diathermy equipment to thereby minimize the introduction of circulating currents therein. While the exact configuration of the strips 28 is not critical it is preferable to maximize the amount of surface area covered by the strips.

The outer end of each of the strips 28 is electrically coupled with an associated axial strip 24. Each strip 28 is preferably coupled with one of the axial strips such that it acts as a continuation of the axial strip to which it is attached. However as shown, three of the strips 28 are connected to one axial strip 24 for rigidity purposes only. The vertical strips 24 and face forming strips 28 may be soldered to each other, be formed in a continuous strip by an etching process or maybe otherwise coupled to provide the electrical connection therebetween. The inner end of each of the strips 28 terminates within the inner area of the surface defined by the face portion of the shield so as to provide an open centre area 30. The strips 28 are positioned to avoid contact with each other to thereby eliminate the "shorted turn" effect. If, for example, two of the strips were in an electrical engagement induced currents in the strips from the magnetic field would result. Power needed to create these induced currents would result in an energy loss which reduced the operating efficiency of the diathermy apparatus.

In use, the electrostatic shield 10 of the present invention is suitably mounted onto the applicator head such that the head's radiating coil 16 is located within the chamber formed by the side and face portion of the shield. In particular, the shield 10 is mounted onto the applicator head such that the side portion 12 of the shield encircles the head's radiating electrode and such that the face portion 14 of the shield is positioned forwardly of the radiating coil in a parallel relationship therewith.

Once the shield 10 is mounted onto the applicator head, RF energy is applied to the radiating coil causing it to radiate electrostatic and electromagnetic energy. The electrostatic energy thus radiated is effectively attenuated by the electrostatic shield 10 while the electromagnetic energy passes through the shield unimpeded. The applicator head is positioned to direct the radiated electromagnetic energy to the body tissues to be treated. Since the side portion 12 of the shield 10 is slotted to provide a plurality of axial side strips 24, the electromagnetic field energy does not produce circulating currents within the side portion of the housing. In addition, the terminating band 26 is positioned sufficiently far away from the radiating coil that the circulating currents produced therein are negligible. In this way, the electrostatic shield of the present invention is capable of effectively isolating the electrostatic energy radiated by radiating coil of the applicator head from external loads without the resultant production of excessive circulating currents within the outer casing of the head or the terminating band of the shield.

**Claim**

A diathermy applicator head for a short wave diathermy apparatus, the head comprising a current carrying radiating coil (16), an electrostatic shield (10) which includes a plurality of electrically conductive first strips (28) which are located so that, in use, they are interposed between the coil (16) and the body of a patient being treated by the coil and a plurality of further conductive strips (24) which surround the coil (16), the first strips (28) extending inwards from the further strips (24), and means for earthing the shield, characterised in that the coil (16) is substantially circular and planar, the first strips (28) extend in a plane substantially parallel to the plane of the coil (16), but leave an open centre area (30), and an electrically conductive loop (26) electrically connects the ends of the further strips (24) remote from the first strips (28), the loop (26) being spaced axially from the coil (16) and on the side thereof remote from the first strips (28) by a distance at least as great as the diameter of the coil (16) so that only negligible electric currents are induced in the loop (26) when current is applied to the coil (16).

**Patentanspruch**

Diathermie-Behandlungskopf für einene Kurzwellen-Diathermieapparat, dessen Kopf eine stromführende Strahlungswicklung (16), eine elektrostatische Abschirmung (10) umfaßt, die eine Vielzahl elektrisch leitender erster Streifen (28) besitzt, die derart angeordnet sind, daß sie im Betrieb zwischen der Wicklung (16) und dem Körper eines durch die Wicklung behandelten Patienten liegen, und einer Vielzahl weiterer leitender Streifen (24), die die Wicklung (16) umgeben, wobei sich die ersten Streifen (28) von den weiteren Streifen (24) nach innen erstrecken, und wobei Mittel zur Erdnung der Abschirmung vorgesehen sind, dadurch gekennzeichnet, daß die Wicklung (16) im wesentlichen kreisförmig und planar ist, und daß sich die ersten Streifen (28) in einer Ebene erstrecken, die im wesentlichen parallel zur Ebene der Wicklung (16) ist, jedoch einen mittigen Bereich (30) offen läßt, und daß eine elektrisch leitende Schleife (26) elektrisch die Enden der weiteren Streifen (24) entfernt von den ersten Streifen (28) verbindet, wobei die Schleife (26) axial von der Wicklung (16) und an ihrer Seite entfernt von den ersten Streifen (28) in einer Entfernung beabstandet ist, die mindestens so groß ist wie der Durchmesser der Wicklung (16), so daß nur vernachlässigbare elektrische Ströme in der Schleife (26) induziert werden, wenn Strom an die Wicklung bzw. Spule (16) angelegt ist.

**Revendication**

Une tête d'applicateur pour diathermie destinée à un appareil de diathermie à ondes courtes, la tête comprenant une bobine d'irradiation (16) parcourue par un courant, un écran électrostatique (10) qui comprend une pluralité de premières bandes (28) conductrices de l'électricité, qui sont placées de manière que pendant l'utilisation elles soient interposées entre la bobine (16) et le corps d'un patient traité par la bobine et une pluralité d'autres bandes conductrices (24) qui entourent la bobine (16), les premières bandes (28) s'étendant vers l'intérieur depuis les autres bandes (24), et un moyen pour mettre l'écran à la terre, caractérisée par le fait que le bobine (16) est sensiblement circulaire et plate, les premières bandes (28) s'étendent dans un plan sensiblement parallèle au plan de la bobine (16) mais laissent une zone centrale ouverte (30), et une boucle (26) conductrice de l'électricité relie électriquement les extrémités des autres bandes (24) éloignées des premières bandes (28), la boucle (26) étant espacée axialement de la bobine (16) et se trouvant sur le côté de celle-ci qui est éloigné des premières bandes (28) d'une distance au moins aussi grande que le diamètre de la bobine (16) de sorte que seuls des courants électriques négligeables sont induits dans la boucle (26) lorsque le courant est appliqué à la bobine (16).

0 038 110

*Fig. 1.*

*Fig. 2.*

1